# EUROPÄISCHE PATENTSCHRIFT

(11) **EP 0 911 063 B1**
(45) Veröffentlichungstag und Bekanntmachung des Hinweises auf die Patenterteilung: **29.12.2004**
(21) Anmeldenummer: 98250370.8
(22) Anmeldetag: 20.10.1998
(51) Int. Cl.: A61N 1/365

(54) **Ratenadaptiver Herzschrittmacher**
Rate adaptive pacemaker
Stimulateur cardiaque à fréquence asservie

(30) Priorität: 23.10.1997 DE 19747820
(43) Veröffentlichungstag der Anmeldung: 28.04.1999
(73) Patentinhaber: BIOTRONIK Mess- und Therapiegeräte GmbH & Co Ingenieurbüro Berlin, 12359 Berlin (DE)
(72) Erfinder: Dauer, Wolfgang, Dipl.-Phys., 91301 Forchheim (DE); Wetzig, Thomas, Dr.-Ing., 81825 München (DE); Fröhlich, Ronald, Dipl.-Phys. Dr., 91056 Erlangen (DE)
(74) Vertreter: Eisenführ, Speiser & Partner

(56) Entgegenhaltungen:
- EP-A- 0 237 767
- EP-A- 0 334 681
- EP-A- 0 607 511
- EP-A- 0 796 636
- US-A- 5 184 615

## Beschreibung

Die Erfindung betrifft einen ratenadaptiven Herzschrittmacher gemäß dem Oberbegriff des Anspruchs 1.

Ratenadaptive Herzschrittmacher, bei denen die Stimulationsfrequenz bzw. -rate in Abhängigkeit von im Körper des Patienten aufgenommenen Signalen eingestellt wird, die den physiologischen Bedarf des Patienten in Bezug auf die Herzaktivität reflektieren, sind seit Jahren in vielgestaltigen Ausführungen bekannt und im klinischen Einsatz. Einen Überblick über die mit der Entwicklung der Ratenadaption in der Schrittmachertechnik verfolgten Ziele und die eingeschlagenen Wege gibt das relativ frühe Standardwerk K. Stangl et al.: Frequenzadaptive Herzschrittmacher, Darmstadt 1990.

Dort (Seiten 255 - 272) wird auch der sogenannte QT-Schrittmacher diskutiert, bei dem die Einstellung der Stimulationsrate anhand der Zeitspanne zwischen einem Stimulationsimpuls und dem Auftreten der T-Zacke des durch den Stimulus initiierten (evozierten) ventrikulären Herzaktionssignals, des sogenannten QT-Intervalls, erfolgt. Dieses Zeitintervall kann zur Ratensteuerung eines Herzschrittmachers herangezogen werden, weil es sowohl von der Herzfrequenz als auch von der Belastung abhängt und die Abhängigkeit von der Herzfrequenz zumindest in einem bestimmten Zeitbereich annähernd linear ist. Nachteilig hierbei ist die aufgrund der geringen Signalamplitude und wenig markanten Signalform in der Praxis recht problematische Erfassung und Auswertung der T-Komponente des intrakardialen EKG und vor allem die Gefahr einer positiven Rückkopplung aufgrund des Umstandes, daß sowohl ein Belastungs- als auch ein Frequenzanstieg zu einer Verkürzung des QT-Intervalls führen.

Aus der EP 0796 636 ist ein ratenadaptiver Herzschrittmacher bekannt, der die Morphologie eines Herzsignalamplitudenverlaufs mittels eines neuronalen Netzwerkes auswertet, um die Morphologie des Herzsignalamplitudenverlaufs zu kategorisieren und in Abhängigkeit davon eine Stimulationsrate abzuleiten.

Gemäß EP 0 232 528 B1 und EP 0 237 767 B1 erfolgt die Steuerung der Stimulationsrate aufgrund eines Vergleiches eines integrierten Herzaktionspotentials mit einem vorab bestimmten Ziel- bzw. Vergleichswert; bei genauerer Betrachtung läuft das Vorgehen auf eine Auswertung der Zeitdauer vom Stimulus bis zum Ende der R-Zacke des ventrikulären EKG ("Depolarisationsgradientendauer") hinaus. Diese Signaldauer ist indes in der Praxis an einem mit Störungen und insbesondere Nullinienschwankungen behafteten Signal schwer verläßlich zu ermitteln.

Der Erfindung liegt deshalb die Aufgabe zugrunde, einen ratenadaptiven Herzschrittmacher der eingangs genannten Gattung mit verbesserter praktischer Brauchbarkeit bereitzustellen

Die Aufgabe wird durch einen Herzschrittmacher mit den Merkmalen des Anspruchs 1 gelöst.

Die Erfindung schließt die technische Lehre ein, charakteristische und auch durch Störungen und Nulliniendrifts nicht wesentlich verfälschte belastungsabhängige Änderungen der Signalmorphologie eines evozierten Herzsignals zur Steuerung der Stimulationsrate heranzuziehen.

Für eine Ratenadaption werden zwei sog. Differenzpunkte (Zeitpunkte ab Stimulus) bestimmt. Aus den Spannungswerten des Herzsignals (speziell der/des sogenannten VER = ventricular evoked response oder ventrikuläre evozierte Reizantwort) an diesen beiden Differenzpunkten wird ein Differenzwert gebildet, der bei physiologischer Schlagfrequenz in einem bekannten Bezug zur Schlagfrequenz steht. Diese Differenz dient als Parameter zur Frequenzanpassung und steigt über die frequenzabhängige Solldifferenz bei zu niedriger Stimulationsfrequenz und sinkt unter die frequenzabhängige Solldifferenz bei zu hoher Stimulationsfrequenz. Die bei einer anfänglichen Kalibration bestimmten Differenzpunkte bleiben konstant und werden Ratenänderungen nicht nachgeführt.

Der erste Differenzpunkt entspricht dem Maximum der T-Welle (T+) bei maximaler Last und angepaßter Stimulationsfrequenz. Als zweiter Differenzpunkt wird der Zeitpunkt gewählt, an dem im Bereich der R+-Welle der größte Unterschied zwischen dem VER-Kurvenverlauf mit maximaler Last bei angepaßter Frequenz und dem VER-Kurvenverlauf in Ruhe bei derselben Frequenz besteht. Zur Kalibration ist die Messung dieser zwei Meßkurven erforderlich.

Eine weitere Verbesserung des Ratenadaptionsalgorithmus läßt sich durch Einbeziehung der aktuellen Signaldauer erreichen. Hierzu wird bei jedem zur Frequenzanpassung verwendeten Signal neben der Signalspannung an den beiden Differenzpunkten zusätzlich (z.B. durch Differentiation) der Zeitpunkt eines charakteristsichen Signalpunktes, etwa der T+-Amplitude, bestimmt. Der Abstand zwischen diesem Zeitpunkt und dem zweiten Differenzpunkt wird als Differenz k ermittelt. Die Berechnung des verfeinerten Ratenadaptionsparameters erfolgt nun vorteilhaft beispielsweise derart, daß die Differenz der Signalspannungen beider Differenzpunkte durch k² dividiert wird.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Darstellung der Belastungsabhängigkeit des VER,
- Figur 2: eine Darstellung der Abhängigkeit des VER von der Stimulationsfrequenz,
- Figur 3: eine Darstellung zur Auswertung der Signalmorphologie des VER für die Ratensteuerung,
- Figur 4: eine Darstellung der Korrelation zwischen Adaptionsparameter und Stimulationsfrequenz bei einer Ausführungsform der Erfindung,
- Figur 5: eine vergleichende Darstellung des zeitlichen Verlaufes der Stimulationsfrequenz sowie eines ersten und eines zweiten Ratenadaptionsparameters,
- Figur 6: eine schematische Darstellung der Steuerung der Stimulationsrate gemäß einer Ausführungsform der Erfindung,
- die Figuren 7a und 7b: Beispiele für bei der Autokalibration des Ratenadaptionalgorithmus benutzte Histogramme,
- die Figuren 8a und 8b: Beispiel-Darstellungen von im Rahmen der Autokalibration relevanten Adaptionsparameter-Frequenz-Abhängigkeiten,
- Figur 9: ein Funktions-Blockschaltbild wesentlicher Komponenten eines ratenadaptiven Herzschrittmachers gemäß einer Ausführungsform der Erfindung und
- Figur 10: ein Funktions-Blockschaltbild eines Herzschrittmachers gemäß einer weiteren Ausführungsform der Erfindung.

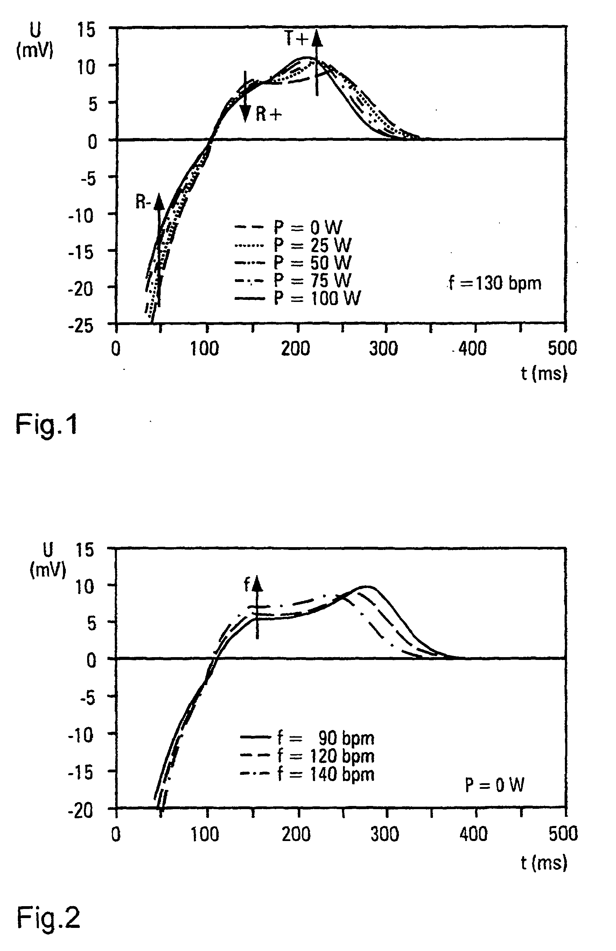

In Fig. 1 ist eine anhand eines Fahrradergometertests bei einer von Ruhe bis auf 100 W gesteigerten Belastung gewonnene Darstellung der Belastungsabhängigkeit der VER-Signalmorphologie gezeigt. Die Stimulationsfrequenz f wurde bei 130 bpm konstant gehalten, und es ist die Signalspannung U über der Zeit t ab dem Stimulusimpuls aufgetragen. An den Kurven ist zu erkennen, daß die Signalspannung im R--Abschnitt sowie im T+-Abschnitt des VER mit wachsender Belastung ansteigt, während sie im R+-Bereich mit zunehmender Belastung abnimmt. Hieraus wird klar, daß eine Auswertung der Signalmorphologie für die Stimulationsratensteuerung vorteilhaft aufgrund eines Vergleiches der Signalamplitude im R-- oder T+-Bereich mit derjenigen im R+-Bereich als möglich erscheint.

Fig. 2 gibt eine Darstellung der Abhängigkeit des VER von der Stimulationsfrequenz im Ruhezustand eines Patienten, wobei wiederum - mit gleicher Skalenteilung wie bei Fig. 1 - die Signalspannung U über der Zeit t ab dem Stimulusimpuls aufgetragen ist. Es wird deutlich, daß die VER-Morphologie auch frequenzabhängig ist.

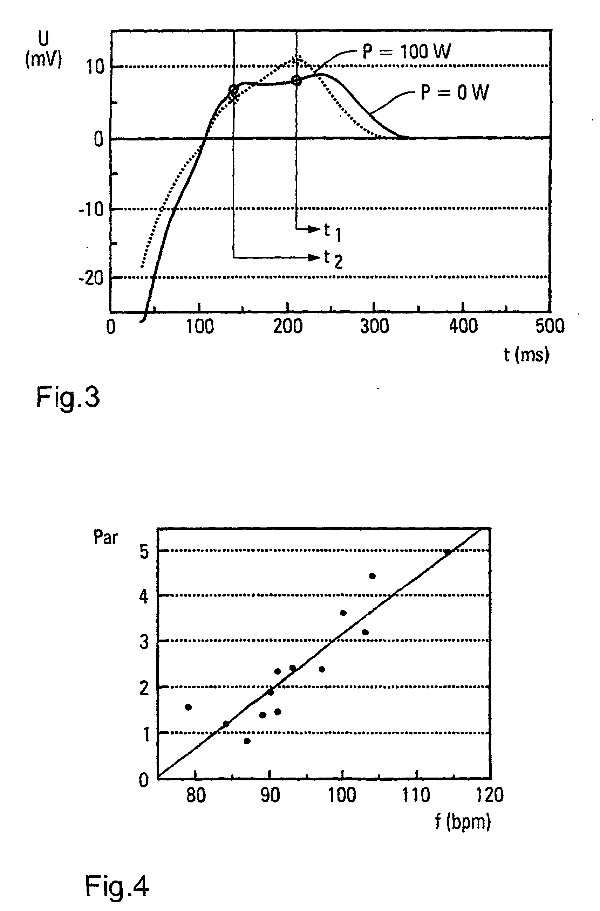

Fig. 3 ist eine Darstellung zur Auswertung der Signalmorphologie des VER für die Ratensteuerung, in der für zwei beispielartige herausgegriffene VER-Signalverläufe zwei Zeitpunkte t₁ und t₂ eingetragen sind, zu denen die Signalamplitude ermittelt wird und die so gewählt sind, daß die Amplitudendifferenz - die als zur Ratendaption verwendeter Parameter Par dient - eine möglichst hohe belastungsabhängige Dynamik aufweist.

Aus der grafischen Darstellung der Korrelation zwischen Adaptionsparameter Par (in willkürlichen Einheiten) und Stimulationsfrequenz f in Fig. 4 wird deutlich, daß der Adaptionsparameter eine annähernd lineare Abhängigkeit aufweist. Die Gerade der linearen Regression veranschaulicht den physiologischen Verlauf. Für den Adaptionsparameter läßt sich mithin ein linearer physiologischer Verlauf angeben. Werte des Parameters oberhalb des physiologischen Verlaufs deuten auf eine Unterstimulation, also auf eine für die derzeitige Belastung zu geringe Stimulationsfrequenz hin. Werte unterhalb des physiologischen Verlauf zeigen eine Überstimulation an. Daher kann durch den Vergleich zwischen dem aktuellen Parameterwert und dem dem physiologischen Verlauf entsprechenden "Sollwert" die Notwendigkeit einer Änderung der Stimulationsfrequenz festgestellt werden.

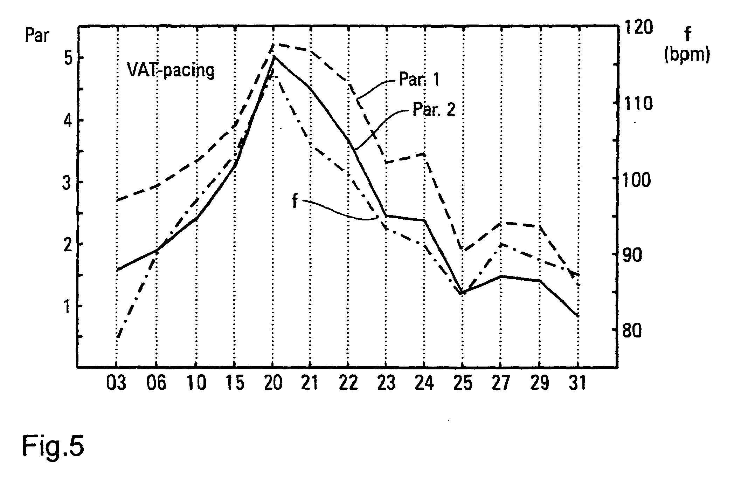

Fig. 5 zeigt in einer vergleichenden Darstellung die zeitlichen Verläufe der Stimulationsfrequenz (strichpunktierte Kurve) sowie eines ersten, gemäß Fig. 4 bestimmten, und eines zweiten, gemäß der weiter oben gegebenen Darstellung unter Einbeziehung der VER-Signalbreite verfeinerten Ratenadaptionsparameters (gestrichelte bzw. durchgezogene Kurve - willkürliche Einheiten) bei einem vorgegebenen Belastungs-Zeit-Programm. Die vergleichende Darstellung zeigt, daß mittels eines aus der VER-Signalmorphologie abgeleiteten Ratensteuerparameters eine gut bis sehr gut angenäherte belastungsabhängige Steuerung der Stimulationsrate möglich ist. Für die Korrelation des ersten Adaptionsparameters mit der Stimulationsfrequenz wurde ein Korrelationskoeffizient von 0,852, für den verfeinerten zweiten Parameter ein Koeffizient von 0,914 ermittelt.

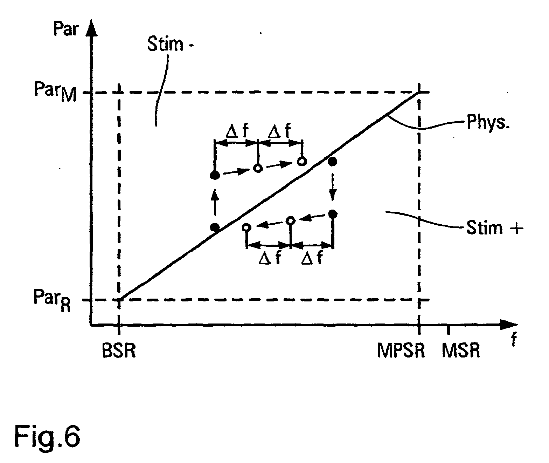

Fig. 6 zeigt in einer schematischen Darstellung eine bevorzugte Art und Weise der Steuerung der Stimulationsrate f in Abhängigkeit von einem gemäß obigen Ausführungen bestimmten Adaptionsparameter Par. Die in der Autokalibration ermittelten Grenzwerte des Adaptionsparameters (nachfolgend auch als "Differenzparameter" bezeichnet) Par für Ruhe (Par_{R}) und für maximale Belastung (Par_{M}) spannen zusammen mit dem vorgegebenen Mindestwert der Stimulationsrate (Basic Sensor Rate = BSR) und der in der Autokalibration ermittelten Maximalrate (Maximum Parameter Sensor Rate = MPSR) eine Fläche auf, innerhalb derer der Differenzparameter für die Frequenzanpassung herangezogen wird. Eine nicht an den Belastungszustand angepaßte Stimulationsfrequenz ist durch eine Abweichung des Differenzparameters vom physiologischen Verlauf gekennzeichnet. Bei der Frequenzanpassung wird eine erhöhte Belastung infolge des damit verbundenen Anstiegs des Differenzparameters in den Bereich der Unterstimulation eindeutig erkannt und mit einer schrittweisen Erhöhung der Stimulationsfrequenz um einen bestimmten Betrag Δf beantwortet, bis der Differenzparameter den physiologischen Verlauf bei höherer Stimulationsfrequenz wieder erreicht hat. Ein analoges Verhalten ergibt sich bei abnehmendem Differenzparameter. Die Schrittweite Δf kann optional an den Betrag der aktuellen Abweichung des Differenzparameters vom physiologischen Verlauf angepaßt werden.

Aufgrund der Tatsache, daß neben Änderungen der VER auch die Stimulationsfrequenz selbst einen begrenzten Einfluß auf den Differenzparameter hat, nähert dieser sich während der schrittweisen Frequenzanpassung in der Darstellung der Fig. 6 in der Regel nicht waagerecht an den physiologischen Verlauf an. Wird beispielsweise in einem ersten Anpassungs-Schritt ein erhöhter Differenzparameter ermittelt, dann wird die Stimulationsfrequenz erhöht. Infolge dieser Erhöhung tritt nun eine zusätzliche Erhöhung des Differenzparameters ein etc. Dieser Frequenzeffekt ist aber innerhalb der durch die Autokalibration festgelegten Grenzen Par_{R} und Par_{M} des Differenzparameters vernachlässigbar klein.

Die Autokalibration erfolgt unter Heranziehung der mit einem zusätzlichen Fühler (Aktivitätsfühler) gewonnenen Belastungssignale. Dabei wird von den folgenden Annahmen ausgegangen:
(a) Der Ruhezustand läßt sich durch statistische Auswertung des Differenzparameters definieren. Hierfür wird ein Histogramm n(Par) erstellt, das die Werte von Par über einen festgelegten Zeitraum (z.B. 1 Woche) enthält.
(b) Das Signal des Aktivitätsfühlers (Motion-Signal = MS) spiegelt die Maximalbelastung gut wider, da diese meist physisch bedingt ist. Aus den Werten des Motion-Signals wird ein Histogramm n(MS) erstellt.

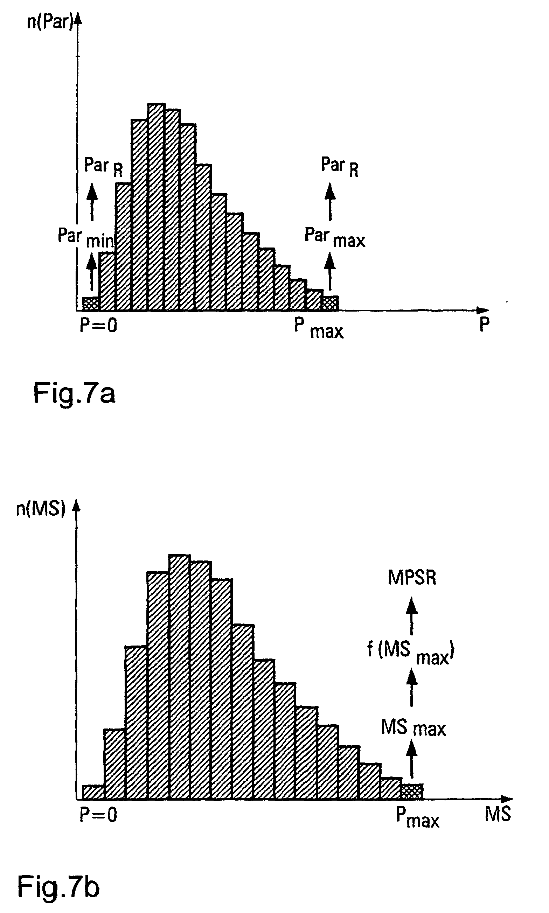

Beispiele für die erwähnten Histogramme sind in Fig. 7a bzw. 7b gezeigt.

Die für die Frequenzanpassung gültigen Frequenzgrenzen BSR und MSR werden vom Arzt festgelegt. Die beiden Differenzpunkte (t₁ bzw. t₂ in Fig. 3) sowie der physiologische Verlauf des Differenzparameters werden in vorgebbaren Zeitabständen automatisch kalibriert, und zwar die Differenzpunkte innerhalb sinnvoller Grenzen derart, daß die belastungsabhängige Dynamik des Differenzparameters maximal ist. Die Bestimmung erfolgt aus dem Vergleich einer in Ruhe und einer bei maximalem Motion-Signal aufgezeichneten VER, die beide während des Betriebs des Schrittmachers ständig aktualisiert werden.

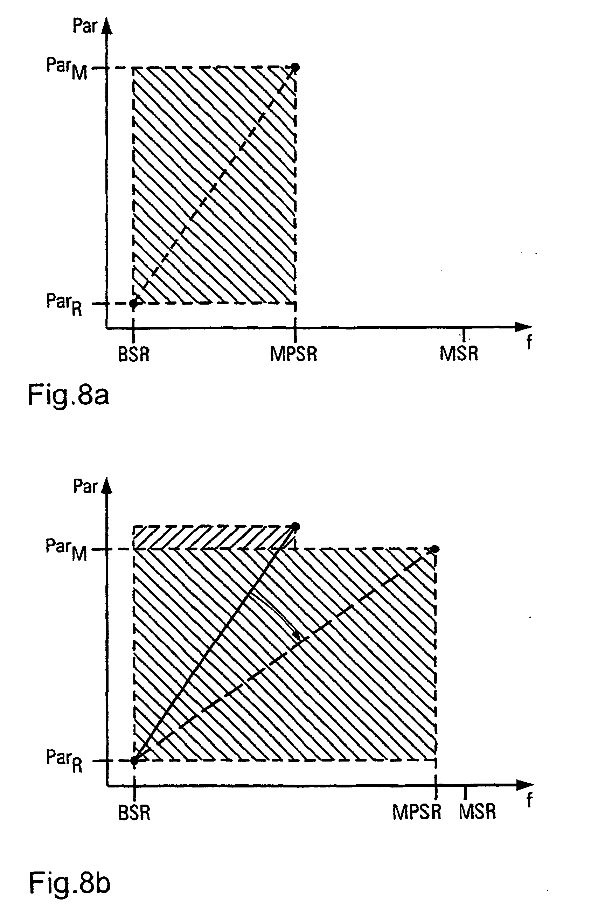

Die Gewinnung der Differenzparameterwerte ist in Fig. 8a und 8b skizziert:

Der Differenzparameter für maximale Belastung (PM) wird anhand der oben erwähnten Histogramme n(Par) und n(MS) ermittelt. Der Ruhewert des Differenzparameters (Par_{R}) wird anhand des Histogramms n(Par) zunächst innerhalb einer initialen Kalibrationsphase kurzfristig (ca. 1 Tag) ermittelt. Im Dauerbetrieb wird er durch die Autokalibration regelmäßig nachgeführt. Dem Parameter PR wird stets die fest vorgegebene Frequenz BSR zugeordnet. Für Par_{M} und MPSR werden willkürliche Anfangswerte gewählt. Par_{M} wird dabei in der Regel oberhalb des im Ergebnis eines längerfristigen Betriebs zu erwartenden Endwerts gewählt, MPSR annähernd in der Mitte des Stimulationsfrequenzbereiches (vgl. Fig. 8a).

Zur Anpassung von Par_{M} und MPSR wird der höchste im Histogramm n(Par) aufgetretene Wert Parₘₐₓ mit dem höchsten im Histogramm n(MS) aufgetretenen Wert MSₘₐₓ korreliert. Pₘₐₓ wird als neues Par_{M} festgelegt, f(MSₘₐₓ) als neues MPSR. Dadurch wird die Fläche, innerhalb derer die VER-Frequenzanpassung benutzt wird, an die höchsten aufgetretenen Werte des Differenzparameters und des Motion-Signals angepaßt (Fig. 8b). Durch dieses Vorgehen besitzt die VER-Frequenzanpassung zunächst eine nur relativ geringe Frequenzdynamik (geringe Empfindlichkeit auf Belastung), die sich jedoch durch die Autokalibration schrittweise erhöht, sobald im Histogramm n(MS) höhere Motion-Signale auftreten.

Bei einer Schrittmacheranordnung in bevorzugter Ausführung der Erfindung ist, wie schon aus den obigen Ausführungen zur Autokalibration deutlich wird, neben Mitteln zur Erfassung der evozierten intrakardialen Potentiale ein zusätzlicher Fühler für eine physiologische bzw. Belastungsgröße vorgesehen. Dessen Einsatz wird durch folgende Regeln bestimmt:
(1) Die kontinuierliche Aufzeichnung des Fühlersignals in Form eines Histogramms n(MS) wird für die Autokalibration herangezogen (siehe oben).
(2) Die Frequenzanpassung wird temporär vom Fühlersignal gesteuert, falls der Differenzparameter den bei der Kalibration festgelegten Wert Par_{M} übersteigt. Eine einfache Extrapolation des physiologischen Verlaufs über die Grenzen Par_{R} und Par_{M} hinaus ist nicht gestattet.
(3) Die Frequenzanpassung wird allein vom Fühlersignal gesteuert, falls die VER unter Belastung keine oder paradoxe Morphologieveränderungen zeigt. Als Kriterium dient eine festgelegte Mindestdynamik des Differenzparameters.
(4) Der Arzt wählt den Fühler unter spezifischen Bedingungen als allein die Ratenadaption bestimmend aus, um etwa unerwünschte medikamentös bedingte Effekte auf die VER-Ratenadaption auszuschließen.

Wie oben bereits kurz angesprochen, kann durch Einbeziehung einer die Signaldauer repräsentierenden Größe (nachfolgend "Signaldauerparameter") das Adaptionsverhalten weiter verbessert werden. Hiermit kann insbesondere vorteilhaft dem sogenannten Orthostase-Effekt Rechnung getragen werden, der in einer Änderung der Signalmorphologie auch bei einer Änderung der Orientierung des Körpers im Raum (Stehen-Liegen usw.) besteht. Der Signaldauerparameter ist z.B. als die Zeitspanne nach der Stimulation bis zum Unterschreiten einer festgelegten Spannungsschwelle gegen Ende der Repolarisationsphase durch die VER definiert. Er hängt sowohl von der Belastung als auch von der Stimulationsfrequenz ab. Die Abhängigkeit von der Last wird durch die Abhängigkeit vom Differenzparameter angenähert. Die Abhängigkeiten vom Differenzparameter und von der Stimulationsfrequenz werden für die aufrechte Körperposition durch Autokalibration getrennt ermittelt und bilden eine zweidimensionale Funktion.

Sobald der Patient von der aufrechten in die waagrechte Körperposition wechselt, steigt der Differenzparameter an. Da der Signaldauerparameter jedoch gleichzeitig unverändert bleibt bzw. sogar leicht ansteigen kann, weicht der Signaldauerparameter unmittelbar von der bekannten Funktion für die aufrechte Körperposition ab. Sobald der Signaldauerparameter um mehr als einen bestimmten Toleranzwert ansteigt, wird die liegende Körperposition erkannt. Beim Wiederaufrichten des Körpers wandert der Signaldauerparameter wieder in den Toleranzbereich.

Zur Korrektur des Orthostase-Effektes wird der physiologische Verlauf des Differenzparameters bei Erkennung einer liegenden Position gegenüber dem Verlauf bei aufrechter Körperposition um einen bestimmten Offset angehoben ("Orthostaseshift"). Dies bewirkt eine Kompensation des Fehlers, der beim Differenzparameter aufgrund des Orthostase-Effekts auftritt, bei gleichzeitiger Aufrechterhaltung des Regelmechanismus. Beim Wechsel in die aufrechte Körperposition wird der Offset natürlich wieder aufgehoben.

Da sich die evozierten Herzsignale nur bei Stimulation ausbilden und intrinsische Signale sich nicht zur vorstehend skizzierten Bestimmung des Adaptionsparameters heranziehen lassen, ist für den kontinuierlichen Frequenzanpassungsbetrieb eine ununterbrochene Stimulation (bei Nutzung des VER eine Ventrikel-Stimulation) auch bei adäquater Eigenfrequenz des Patienten zu gewährleisten. Darüber hinaus muß wegen der damit verbundenen unphysiologischen Beeinflussung des Differenzparameters das Auftreten von Fusion-Beats (nahezu gleichzeitiges Auftreten von Stimulationsimpuls und spontaner Herzaktion) vermieden werden.

Im DDD-Modus bestehen grundsätzlich folgende Möglichkeiten:
A1) Die AV-Zeit des Schrittmachers wird generell so kurz eingestellt, daß die ventrikuläre Stimulation sichergestellt ist. Daraus resultiert u.U. jedoch eine unphysiologisch niedrige AV-Zeit.
A2) Die AV-Zeit wird an einen Wert knapp unter dem physiologischen Wert angepaßt. Hierzu wird sie in bestimmten Zeitabständen kurzzeitig um einen bestimmten Betrag verlängert. Wird der Schrittmacher bei verlängerter AV-Zeit aufgrund einer Eigenaktion des Ventrikels inhibiert, wird sie daraufhin unter den ursprünglichen Wert verringert, um die Gefahr von Fusion-Beats zu verringern. Anderenfalls wird der ursprünglich eingestellte Wert weiterbenutzt.
A3) Die AV-Zeit wird alle x Schläge für y Schläge um einen bestimmten Betrag verringert, so daß für diese y Schläge die ventrikuläre Stimulation und somit die Ermittlung des Adaptionsparameters sichergestellt ist.

Im VVI-Modus bestehen folgende Möglichkeiten:
B1) Bei ventrikulärer Eigenaktion wird die Stimulationsfrequenz alle x Schläge für y Schläge geringfügig über die Eigenfrequenz angehoben, um den Differenzparameter in regelmäßigen Zeitabständen ermitteln zu können. Nachteilig dabei ist die verlängerte Reaktionszeit der Ratenanpassung.
B2) Bei ventrikulärer Eigenaktion wird die Stimulationsfrequenz generell anstatt durch den Differenzparameter durch das Signal des zusätzlichen Fühlers gesteuert.

Auch bei zweckmäßiger Nutzung der Möglichkeiten A3) oder B1) kann der Fall eintreten, daß zeitweilig der Adaptionsparameter nicht aktuell zur Verfügung steht. Um eine Reduzierung der Ansprechzeit des Ratenadaptionsalgorithmus zu erreichen, wird während dieser Zeiträume bevorzugt eine in der jüngeren Vergangenheit festgestellte Tendenz der Stimulationsrate fortgeschrieben. Wenn also beispielsweise im letzten Zeitraum der Verfügbarkeit des Adaptionsparameters ein Anstieg der Stimulationsrate verzeichnet wurde, wird die Stimulationsrate auch bei fehlenden aktuellen Parameterwerten weiter angehoben.

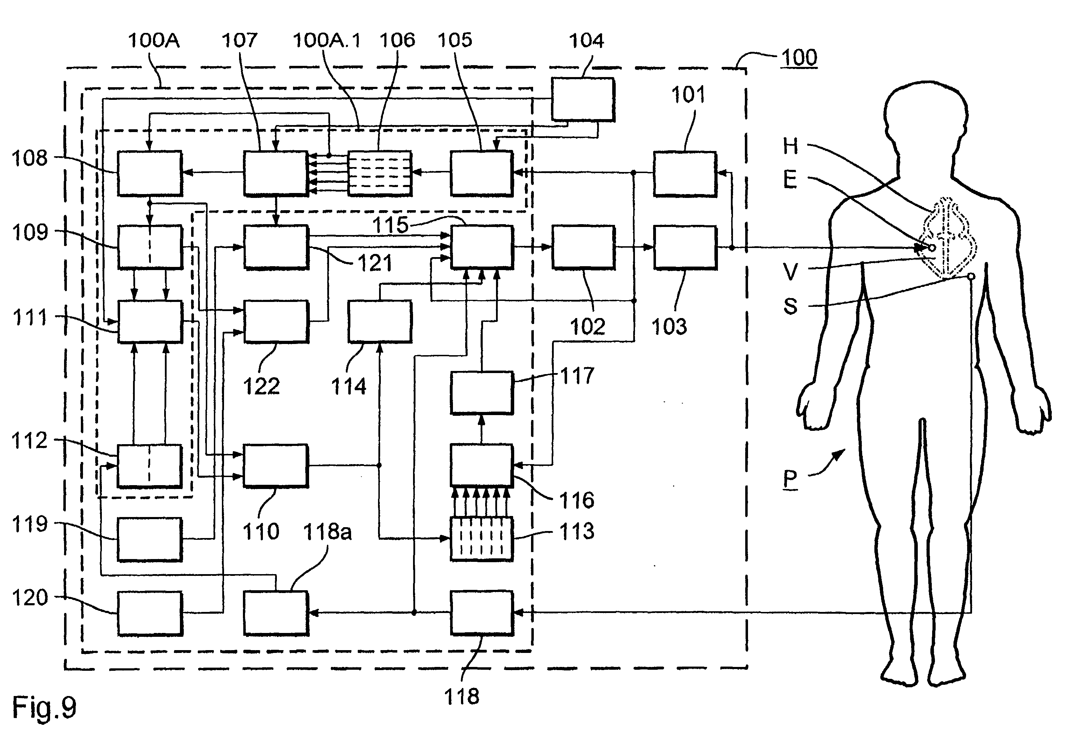

In Fig. 9 sind in Form eines Funktions-Blockschaltbildes wesentliche Komponenten eines ratenadaptiven Herzschrittmachers 100 gezeigt, wobei im Interesse der Übersichtlichkeit an sich bekannte, für die Ausführung der Erfindung jedoch nicht wesentliche Schrittmacherelemente fortgelassen sind.

Der Herzschrittmacher 100 ist eingangsseitig mit einer intrakardialen Elektrode E im Ventrikel V des Herzens H eines Patienten P und einem Aktivitätsfühler S und zugleich ausgangsseitig mit der Elektrode E verbunden. Über die Elektrode E erfasste ventrikuläre Herzsignale werden dem Eingang einer zur signalformgetreuen Erfassung intrakardialer EKG-Signale ausgebildeten (als solche bekannten) Herzsignal-Eingangsstufe 101 zugeführt, und durch einen Stimulationsimpulsgenerator 102 erzeugte Stimulationsimpulse werden über eine Ausgangsstufe 103 und die Elektrode E an den Ventrikel V abgegeben. Der Schrittmacher 100 enthält eine Ablaufsteuerung 104, die den gesamten Betriebsablauf steuert - in der Figur sind aber im Interesse der Übersichtlichkeit nur die Steuersignalverbindungen zu einigen im gegebenen Zusammenhang wichtigen Komponenten gezeigt.

Die Erzeugung und Bereitstellung der Ratensteuersignale zur Steuerung der Stimulationsrate entsprechend dem physiologischen Bedarf des Patienten P erfolgt in einer eingangsseitig sowohl mit der Herzsignal-Eingangsstufe 101 als auch dem Aktivitätsfühler S und ausgangsseitig mit dem Stimulationsimpulsgenerator 102 verbundenen Ratenberechnungseinrichtung 100A, die als für die Ausführung der Erfindung wesentlichsten Funktions-Block eine Signalamplituden-Verarbeitungseinheit 100A.1 aufweist.

Die Signalamplituden-Verarbeitungseinheit 100A.1 umfasst eine über eine (ihrerseits durch die Ablaufsteuerung 104 getriggerte) A/D-Wandler- und Speicherzugriffseinheit 105 mit der Herzsignal-Eingangsstufe 101 verbundene serielle Herzsignal-Speichereinrichtung 106 mit einer Mehrzahl von Speicherbereichen für jeweils eine vollständige evozierte ventrikuläre Herzaktion (VER). Mit dem Ausgang der Speichereinrichtung 106 sind eine - ebenfalls durch die Ablaufsteuerung 104 gesteuerte Variations-Auswertungseinheit 107 und eine Subtraktionsstufe 108 verbunden. In der Variations-Auswertungseinheit 107 werden für einen vorbestimmten Zeitraum oder eine vorbestimmte Anzahl von evozierten Herzsignalen (entsprechend einem in der Ablaufsteuerung 104 gespeicherten Programm einmalig oder in vorbestimmten größeren Abständen diejenigen Abschnitte bzw. Zeitpunkte des VER-Signals ermittelt, für die die Differenz der Signalamplituden maximale Variabilität zeigt. In der Subtraktionsstufe 108 wird fortlaufend für das jeweils letzte aufgenommene Herzsignat U(t) der aktuelle Amplitudendifferenzwert ΔU = U2(t2) - U1(t1) zu diesen beiden Zyklus-Zeitpunkten (oben als Adaptions- oder Differenzparameter bezeichnet) gebildet. Ausgangsseitig ist die Subtraktionsstufe mit einem Amplitudendifferenzspeicher 109 sowie einer Umrechnungsstufe 110 verbunden. Im Amplitudendifferenzspeicher 109 werden - fortlaufend aktualisiert - der jeweils erfaßte größte und kleinste Amplitudendifferenzwert festgehalten und einem Eingangspaar einer Normierungsstufe 111 zugeführt, die über ein zweites Eingangspaar mit einem Ratengrenzwertspeicher 112 verbunden ist, in dem die für die Ratensteuerung minimal und maximal zulässige Stimulationsrate gespeichert sind.

In der Normierungsstufe 111 wird aus dem jeweils vorliegenden Amplitudendifferenz-Minimal- und -Maximalwert und dem Stimulationsraten-Minimal- und - Maximalwert der aktuell gültige physiologische Verlauf des Differenzparameters in Abhängigkeit von der Stimulationsrate ermittelt und der Umrechnungsstufe 110 zugeführt. In letzterer wird hieraus und aus dem aus der Subtraktionsstufe 108 erhaltenen aktuellen Wert des Differenzparameters gemäß dem oben erläuterten Vorgehen das aktuelle Ratensteuersignal errechnet.

Die Umrechnungsstufe 110 ist ausgangsseitig parallel mit einem Ratensteuersignalspeicher 113 sowie einer Glättungsstufe 114 verbunden. Die Glättungsstufe 114, in der (auf an sich bekannte Weise) eine Nachverarbeitung des Ratensteuersignals zur Vermeidung zu großer "Ratensprünge" erfolgt, ist mit einem Eingang einer Umschalteinheit 115 verbunden, über die das Ratensteuersignal unter den nachfolgend angegebenen Bedingungen letztlich zum Stimulationsimpulsgenerator 102 durchgeschaltet wird.

Der Ratensteuersignalspeicher 113 ist mit dem Eingang einer Trendberechnungsstufe 116 verbunden, die über eine Steuersignalverbindung mit der Herzsignal-Eingangsstufe 101 verbunden ist und über diese aktiviert wird, falls kein VER-Signal erfaßt wird. Die Trendberechnungsstufe 116 lädt nach ihrer Aktivierung die gespeicherten Ratensteuersignale in zeitlicher Zuordnung und führt hierfür eine Trendberechnung durch, deren Ergebnis an eine Ersatzsteuersignal-Berechnungseinheit 117 ausgegeben wird. Diese berechnet ihrerseits in Fortschreibung des errechneten Trends ein Ersatz-Ratensteuersignal. Dieses wird durch die - ebenfalls über die Herzsignal-Eingangsstufe 101 aktivierte - Umschalteinheit 115 zur Ersetzung des bei Fehlen eines VER-Signals nicht verfügbaren originären Ratensteuersignals zum Stimulationsimpulsgenerator durchgeschaltet.

Der Aktivitätsfühler S ist mit einer Fühlersignal-Verarbeitungseinheit 118 verbunden, die - gemäß bekannten Algorithmen - aus dem Aktivitätssignal ein weiteres Ersatz-Ratensteuersignal berechnet, das an einem weiteren Eingang der Umschalteinheit 115 bereitgestellt wird. Zudem bildet die Fühlersignal-Verarbeitungseinheit 118 den jeweils gültigen Stimulationsraten-Maximalwert und übergibt diesen an den Ratengrenzwertspeicher 112.

Die Ratenberechnungseinrichtung 100A umfasst weiterhin einen Variationsbreitenspeicher 119 und einen Amplitudendifferenz-Maximalwertspeicher 120, in denen als für den Anwendungsbereich der vorgeschlagenen Lösung wesentliche Randbedingungen einerseits ein minimal zulässiger Amplitudenvaribilitätswert und andererseits der maximal zulässige Amplitudendifferenz- bzw. Differenzparameterwert gespeichert sind. Die gespeicherten Werte werden in einer Variabilitäts-Vergleichereinheit 121 bzw. einer Amplitudendifferenz-Vergleichereinheit 122 einem Vergleich mit den jeweiligen Ist-Werten unterzogen, die den Vergleichereinheiten 121, 122 von der Variations-Auswertungseinheit 107 bzw. vom Amplitudendifferenzspeicher 109 zugeführt werden. Ergibt der Vergleich, dass eine der geforderten Randbedingungen nicht erfüllt ist, wird durch Ausgabe eines entsprechenden Steuersignals durch die Vergleichereinheiten 121, 122 an die Umschalteinheit 115 anstelle des aufgrund der Signalmorphologie errechneten Ratensteuersignals das durch die Fühlersignal-Verarbeitungseinheit 118 erzeugte Ersatz-Ratensteuersignal an den Stimulationsimpulsgenerator durchgeschaltet.

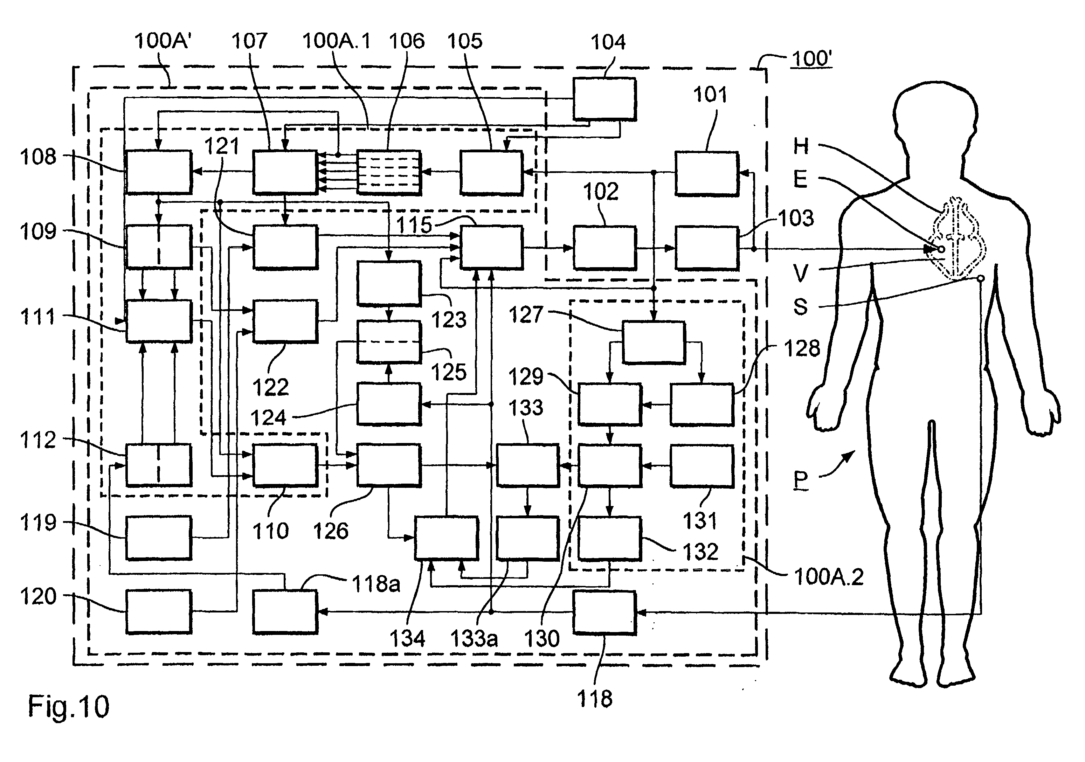

In Fig. 10 ist schematisch ein gegenüber der Ausführung nach Fig. 9 modifizierter Herzschrittmacher 100' dargestellt. Die mit Fig. 9 übereinstimmenden Funktionskomponenten sind mit den gleichen Ziffern wie dort bezeichnet und werden hier nicht nochmals erläutert.

Eine erste Gruppe zusätzlicher Komponenten umfasst einen mit dem Eingang der Subtraktionsstufe 108 verbundenen Langzeit-Amplitudendifferenzspeicher 123, einen mit dem Ausgang der Fühlersignal-Verarbeitungseinheit 118 verbundenen Langzeit-Fühlersignalspeicher 124 und eine mit beiden Langzeitspeichern verbundene mehrstufige Statistik-Auswertungseinrichtung 125 zur Bildung eines Amplitudendifferenz- und eines Fühlersignal-Histogramms und zur Herstellung einer Korrelation zwischen beiden Histogrammen, wobei das Ratensteuersignal in Abhängigkeit von der hergestellten Korrelation erzeugt wird. Eine Ratensteuersignal-Ausgabestufe 126 liefert letztlich das zur Steuerung des Stimulationsimpulsgenerators 102 benötigte Ratensteuersignal aufgrund der Auswertung der Signalmorphologie. Dieses wird dem Impulsgenerator über die Umschalteinheit 115, deren grundsätzliche Funktion oben unter Bezugnahme auf Fig. 9 erläutert wurde, zugeführt.

Der Herzsignal-Eingangsstufe 101 sind Signalbreiten-Erfassungsmittel 127 zur Erfassung der aktuellen Herzsignalbreite nachgeschaltet, und die Ratenberechungseinrichtung 100A weist zusätzlich eine Signalbreiten-Verarbeitungseinheit 100A.2 zur Erzeugung des Ratensteuersignals unter Berücksichtigung der Herzsignalbreite auf.

Die Signalbreiten-Verarbeitungseinheit 100A.2 umfaßt einen mit den Signalbreiten-Erfassungsmitteln 127 verbundenen Signalbreitenspeicher 128, eine mit den Signalbreiten-Erfassungsmitteln 127 und dem Signalbreitenspeicher 128 verbundene Berechnungseinheit 129, einen Referenzwertspeicher 131 und eine mit der Berechnungseinheit 129 und dem Referenzwertspeicher 131 verbundene Vergleichereinheit 130 zum Vergleich einer von der aktuellen Signalbreite abhängigen Größe mit einem Referenzwert. Mit den Ausgängen der Signalamplituden-Verarbeitungseinheit 100A.1 und der Signalbreiten-Verarbeitungseinheit 100A.2 ist ein UND-Gatter 133 verbunden, welches die direkte Ausgabe des durch die Signalamplituden-Verarbeitungseinheit 100A.1 erzeugten Ratensteuersignals nur dann erlaubt, wenn das Vergleichsergebnis eine vorbestimmte Eigenschaft hat, speziell wenn die Signalbreite einen bestimmten Wert nicht unterschreitet.

Die Signalbreiten-Verarbeitungseinheit 100A.2 weist überdies eine Korrekturwert-Berechnungsstufe 132 auf, deren Ausgang zusammen mit dem Ausgang der Signalamplituden-Verarbeitungseinheit 100A.1 auf eine Korrekturstufe 134 geführt ist, die über einen Inverter 133a mit dem Ausgang des UND-Gatters 133 verbunden ist. Wenn das Vergleichsergebnis die genannte vorbestimmte Eigenschaft nicht hat, wird eine Korrektur der durch die Signalamplituden-Verarbeitungseinheit 100A.1 erzeugten Ratensteuersignale vorgenommen.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Ratenadaptiver Herzschrittmacher (100) mit
einem Stimulationsimpulsgenerator (102) und mit diesem verbundener Ausgangsstufe (103) zur Stimulation mindestens des Ventrikels des Herzens
einer Herzsignal-Eingangsstufe (101) zur Signalformerfassung evozierter Herzsignale, insbesondere der Morphologie der ventrikulären evozierten Reizantwort VER,
einer eingangsseitig mit der Herzsignal-Eingangsstufe (101) und ausgangsseitig mit dem Stimulationsimpulsgenerator verbundenen Ratenberechnungseinrichtung (100A) zur Berechnung der Stimulationsrate entsprechend der physiologischen Belastung des Herzschrittmacherträgers und zur Ausgabe eines Ratensteuersignals,
wobei die Ratenberechnungseinrichtung (100A) das Ratensteuersignal aufgrund der von der Eingangsstufe erfassten Herzsignalform erzeugt,
**dadurch gekennzeichnet, dass**
die Ratenberechungseinrichtung (100A) eine Signalamplituden-Verarbeitungseinheit (100A.1) zur Berechnung des Ratensteuersignals im Ansprechen auf eine Herzsignalamplitudendifferenz zwischen zwei Herzsignalamplitudenwerten zu zwei vorbestimmten Zyklus-Zeitpunkten eines vorgegebenen, insbesondere des letzten vorangegangenen, Herzzyklus aufweist.

2. Ratenadaptiver Herzschrittmacher nach Anspruch 1, **dadurch gekennzeichnet, dass** die Signalamplituden-Verarbeitungseinheit (100A.1) eine Subtraktionsstufe (108) zur Ermittlung der Herzsignal-Amplitudendifferenz zu zwei vorbestimmten Zyklus-Zeitpunkten aufweist.

3. Ratenadaptiver Herzschrittmacher nach Anspruch 2, **dadurch gekennzeichnet, dass** die Signalamplituden-Verarbeitungseinheit (100A.1) eine Herzsignal-Speichereinrichtung (106) zur Speicherung einer Mehrzahl von Herzsignalverläufen und eine mit der Herzsignal-Speichereinrichtung (106) verbundene Variations-Auswertungseinheit (107) aufweist, die für einen vorbestimmten Zeitraum oder eine vorbestimmte Anzahl von evozierten Herzsignalen die zwei Zyklus-Zeitpunkte des Herzsignals ermittelt, für die die Differenz der Herzsignalamplituden maximale Variabilität zeigt.

4. Ratenadaptiver Herzschrittmacher nach Anspruch 3, **dadurch gekennzeichnet, dass** die Ratenberechnungseinrichtung (100A) einen Ratengrenzwertspeicher (112) für einen unteren und einen oberen Grenzwert der Stimulationsrate, Amplitudendifferenz-Speichermittel (120) für eine minimale und eine maximale Herzsignalamplitudendifferenz und eine mit den Stimulationsraten- und Amplitudendifferenz-Speichermitteln verbundene Normierungsstufe (111) aufweist, welche für den vorbestimmten Zeitraum oder die vorbestimmte Anzahl von Herzsignalen eine physiologisch normierte Abhängigkeit der Herzsignalamplitudendifferenz von der adaptierten Stimulationsrate ermittelt, indem sie die gespeicherte minimale und maximale Herzsignalamplitudendifferenz mit dem gespeicherten unteren bzw. oberen Grenzwert für die Stimulationsrate verknüpft, und welche das Ratensteuersignal aus der Abweichung der aktuell ermittelten Herzsignalamplitudendifferenz von der physiologisch normierten Abhängigkeit ableitet.

5. Ratenadaptiver Herzschrittmacher nach einem der vorhergehenden Ansprüche, **dadurch gekennzeichnet, dass** die Herzsignal-Eingangsstufe (101) zur Erfassung mindestens der R+-Welle der VER und die Ratenberechnungseinrichtung (100A) zur Erzeugung des Ratensteuersignals im Ansprechen auf eine in diesem Herzsignalabschnitt ermittelte Herzsignalamplitudendifferenz ausgebildet sind.

6. Ratenadaptiver Herzschrittmacher nach einem der vorhergehenden Ansprüche, **dadurch gekennzeichnet, dass** die vorbestimmten Zyklus-Zeitpunkte invariant gegenüber Änderungen der Stimulationsrate gehalten werden.

7. Ratenadaptiver Herzschrittmacher nach einem der Ansprüche 4 bis 6, **dadurch gekennzeichnet, dass** die Ratenberechnungseinrichtung (100A) eine Glättungsstufe aufweist, die im Ansprechen auf eine einen vorbestimmten Betrag überschreitende Abweichung der aktuell ermittelten Herzsignalamplitudendifferenz von der physiologisch normierten Abhängigkeit ein eine stufenweise Anpassung der Stimulationsrate bewirkendes Ratensteuersignal ausgibt.

8. Ratenadaptiver Herzschrittmacher nach einem der vorhergehenden Ansprüche, **dadurch gekennzeichnet, dass** ein zusätzlicher, mit einem Eingang der Ratenberechnungseinrichtung (100A) verbundener Aktivitätsfühler (S) zur Erfassung eines von der physiologischen Belastung abhängigen Parameters vorgesehen ist und die Ratenberechnungseinrichtung (100A) Mittel zur Berücksichtigung des Fühlersignals bei der Erzeugung des Ratensteuersignals aufweist.

9. Ratenadaptiver Herzschrittmacher nach Anspruch 8, **dadurch gekennzeichnet, dass** die Ratenberechnungseinrichtung (100A) eine Fühlersignal-Verarbeitungseinheit (118) aufweist, die für den vorbestimmten Zeitraum oder die vorbestimmte Anzahl von Herzzyklen aufgrund des aufgetretenen Maximalwertes des physiologischen Parameters den oberen Grenzwert für die Stimulationsrate bestimmt.

10. Ratenadaptiver Herzschrittmacher nach Anspruch 8 oder 9, **dadurch gekennzeichnet, dass** die Ratenberechnungseinrichtung (100A) ein Amplitudendifferenz-Speichermittel (120) zur Speicherung eines für die Stimulationsratenanpassung aufgrund der Herzsignalform maximal zulässigen Amplitudendifferenzwertes, eine mit dem Amplitudendifferenz-Speichermittel (120) verbundene Amplitudendifferenz-Vergleichereinheit (122) zum Vergleich des aktuell ermittelten Amplitudendifferenzwertes mit dem gespeicherten Amplitudendifferenz-Maximalwert und eine über den Ausgang der Amplitudendifferenz-Vergleichereinheit steuerbare Umschalteinheit (115) zur Übermittlung eines aufgrund des Fühlersignals berechneten Ersatz-Ratensteuersignals an den Stimulationsimpulsgenerator (102) bei Überschreitung des maximalen Amplitudendifferenzwertes aufweist.

11. Ratenadaptiver Herzschrittmacher nach einem der Ansprüche 8 bis 10, **dadurch gekennzeichnet, dass** die Ratenberechnungseinrichtung (100A) einen Variationsbreitenspeicher (119) zur Speicherung eines für die Stimulationsratenanpassung aufgrund der Herzsignalform minimal zulässigen Amplitudenvariabilitätswertes, eine mit dem Variationsbreitenspeicher (119) verbundene Variabilitäts-Vergleichereinheit (121) zum Vergleich des aktuell ermittelten Amplitudenvariabilitätswertes mit dem gespeicherten Amplitudenvariabilitäts-Minimalwert und eine über den Ausgang der Variabilitäts-Vergleichereinheit (121) steuerbare Umschalteinheit (115) zur Übermittlung eines aufgrund des Fühlersignals berechneten Ersatz-Ratensteuersignals an den Stimulationsimpulsgenerator (102) bei Unterschreitung des minimalen Amplitudenvariabilitätswertes aufweist.

12. Ratenadaptiver Herzschrittmacher nach einem der Ansprüche 8 bis 11, **dadurch gekennzeichnet, dass** die Ratenberechnungseinrichtung einen Langzeit-Amplitudendifferenzspeicher (123), einen Langzeit-Fühlersignalspeicher (124) und eine mit beiden Langzeitspeichern (123, 124) verbundene mehrstufige Statistik-Auswertungseinrichtung (125) zur Bildung eines Amplitudendifferenz- und eines Fühlersignal-Histogramms und zur Herstellung einer Korrelation zwischen beiden Histogrammen auf weist, wobei das Ratensteuersignal in Abhängigkeit von der hergestellten Korrelation erzeugt wird.

13. Ratenadaptiver Herzschrittmacher nach einem der vorhergehenden Ansprüche, **dadurch gekennzeichnet, dass** die Ratenberechnungseinrichtung (100A) einen Amplitudendifferenz- oder einen Ratensteuersignalspeicher (113), eine mit dem Amplitudendifferenz- oder Ratensteuersignalspeicher (113) verbundene Trendberechnungsstufe (116) und eine mit dem Ausgang der Trendberechnungsstufe (116) verbundene Ersatzsteuersignal-Berechnungs-Einheit (117) sowie eine eingangsseitig mit dem Ausgang des Stimulationsimpulsgenerators (102) und/oder der Herzsignal-Eingangsstufe (101) verbundene Umschalteinheit (115) aufweist derart, dass bei Inhibierung des Stimulationsimpulsgenerators bzw. Nichterfassung eines evozierten Herzsignals das Ratensteuersignal durch die Ersatzsteuersignal-Berechnungseinheit (117) in Fortschreibung des durch die Trendberechnungsstufe (116) ermittelten Amplitudendifferenz- oder Ratensteuersignaltrends erzeugt wird.

14. Ratenadaptiver Herzschrittmacher nach einem der vorhergehenden Ansprüche, **dadurch gekennzeichnet, dass** der Herzsignal-Eingangsstufe (101) Signalbreiten-Erfassungsmittel (127) zur Erfassung der aktuellen Herzsignalbreite, insbesondere beginnend mit der Abgabe eines Stimulationsimpulses oder mit dem Überschreiten einer vorgegebenen Signalanfangs-Amplitude und endend mit dem Unterschreiten einer vorgegebenen Signalend-Amplitude, nachgeschaltet sind und die Ratenberechungseinrichtung (100A) eine Signalbreiten-Verarbeitungseinheit (100A.2) zur Erzeugung des Ratensteuersignals unter Berücksichtigung der Herzsignalbreite aufweist.

15. Ratenadaptiver Herzschrittmacher nach Anspruch 14, **dadurch gekennzeichnet, dass** die Signalbreiten-Verarbeitungseinheit (100A.2) einen mit den Signalbreiten-Erfassungsmitteln (127) verbundenen Signalbreitenspeicher (128), eine mit den Signalbreiten-Erfassungsmitteln (127) und dem Signalbreitenspeicher (128) verbundene Berechnungseinheit (129), einen Referenzwertspeicher (131) und eine mit der Berechnungseinheit (129) und dem Referenzwertspeicher (131) verbundene Vergleichereinheit (130) zum Vergleich einer von der aktuellen Signalbreite abhängigen Größe mit einem Referenzwert aufweist und mit den Ausgängen der Signalamplituden-Verarbeitungseinheit (100A.1) und der Signalbreiten-Verarbeitungseinheit (100A.2) ein UND-Gatter (133) verbunden ist, welches die direkte Ausgabe des durch die Signalamplituden-Verarbeitungseinheit (100A.1) erzeugten Ratensteuersignals nur dann erlaubt, wenn das Vergleichsergebnis eine vorbestimmte Eigenschaft hat.

16. Ratenadaptiver Herzschrittmacher nach Anspruch 15, **dadurch gekennzeichnet, dass** die Signalbreiten-Verarbeitungseinheit (100A.2) eine Korrekturwert-Berechnungsstufe (132) aufweist, deren Ausgang zusammen mit dem Ausgang der Signalamplituden-Verarbeitungseinheit (100A.1) auf eine Korrekturstufe (134) geführt ist, die über einen Inverter (133a) mit dem Ausgang des UND-Gatters (133) verbunden ist derart, dass, wenn das Vergleichsergebnis die vorbestimmte Eigenschaft nicht hat, eine Korrektur der durch die Signalamplituden-Verarbeitungseinheit (100A.1) erzeugten Ratensteuersignals vorgenommen wird.

## Claims

1. Rate-adaptive pacemaker (100) comprising a stimulation pulse generator (102) and output stage (103) connected thereto for stimulating at least the ventricle of the heart, a heart-signal input stage (101) for detecting the shape of evoked heart signals, in particular the morphology of the ventricular evoked response VER, a rate calculation device (100A), connected at the input side to the heart-signal input stage (101) and at the output side to the stimulation pulse generator, for calculating the stimulation rate based on physiological stress of an individual in whom the pacemaker is implanted and for outputting a rate-control signal, the rate calculation device (100A) generating the rate-control signal on the basis of the heart signal shape detected by the input stage, **characterised in that** the rate calculation device (100A) comprises a signal-amplitude processing unit (100A.1) for calculating the rate-control signal in response to heart a signal amplitude difference between two heart signal amplitude values at two predetermined times during a specified heart cycle, in particular the immediately preceding heart cycle.

2. Rate-adaptive pacemaker according to claim 1, **characterised in that** the signal-amplitude processing unit (100A.1) comprises a subtraction stage (108) for determining the heart-signal amplitude difference at two predetermined points in time during the cycle.

3. Rate-adaptive pacemaker according to claim 2, **characterised in that** the signal-amplitude processing unit (100A.1) comprises a heart signal memory (106) for storing a plurality of heart signal curves, and a variation analysis unit (107) connected to the heart signal memory (106) and determining for a predetermined time period or a predetermined number of evoked heart signals the two cycle times of the heart signal for which the difference in heart signal amplitudes shows maximum variability.

4. Rate-adaptive pacemaker according to claim 3, **characterised in that** the rate calculation device (100A) comprises a rate threshold memory (112) for a lower and an upper threshold of the stimulation rate, amplitude difference storage means (120) for a minimum and a maximum heart signal amplitude difference and a scaling stage (111) connected to the stimulation rate and amplitude difference storage means, the scaling stage determining a physiologically standardised dependence of the heart signal amplitude difference on the adapted stimulation rate for the predetermined time period or the predetermined number of heart cycles, **in that** it links the stored minimum and maximum heart signal amplitude difference values with the stored lower and upper thresholds for the stimulation rate and derives the rate-control signal based on a deviation between the actually determined heart-signal amplitude difference and the physiologically scaled dependence.

5. Rate-adaptive pacemaker according to any one of the preceding claims, **characterised in that** the heart signal input stage (101) is arranged to detect at least the R+ wave of the VER and the rate calculation device (100A) is arranged to generate the rate-control signal in response to a heart signal amplitude difference detected in this heart signal segment.

6. Rate-adaptive pacemaker according to any one of the preceding claims, **characterised in that** the predetermined cycle times are kept invariant relative to changes in the stimulation rate.

7. Rate-adaptive pacemaker according to any one of claims 4 to 6, **characterised in that** the rate calculation device (100A) comprises a smoothing stage, the smoothing stage emitting a rate-control signal that effects a stage-by-stage adaptation of the stimulation rate in response to a deviation in the actually determined heart signal amplitude difference from the physiologically standardised dependence exceeding a predetermined amount.

8. Rate-adaptive pacemaker according to any one of the preceding claims, **characterised in that** an additional activity sensor (S), connected to an input of the rate calculation device (100A) for detecting a parameter that depends on the physiological stress is provided, and the rate calculation device (100A) comprises means for taking into account the sensor signal when generating the rate-control signal.

9. Rate-adaptive pacemaker according to claim 8, **characterised in that** the rate calculation device (100A) comprises a sensor signal processing unit (118), which determines the upper threshold for the stimulation rate for the predetermined time period or the predetermined number of heart cycles based on a maximum value that has occurred for the physiological parameter.

10. Rate-adaptive pacemaker according to either claim 8 or claim 9, **characterised in that** the rate calculation device (100A) comprises an amplitude difference memory (120) for storing a maximum permissible amplitude difference value for stimulation rate adaptation based on heart signal shape, an amplitude difference comparison unit (122) connected to the amplitude difference memory (120) for comparing the actually determined amplitude difference value to the stored maximum amplitude difference value, and a switching unit (115) controllable by the output of the amplitude difference comparison unit to transmit a substitute rate-control signal to the stimulation pulse generator (102) if the maximum amplitude difference value is exceeded, the substitute rate-control signal being calculated based on the sensor signal.

11. Rate-adaptive pacemaker according to any one of claims 8 to 10, **characterised in that** the rate calculation device (100A) comprises a variation width memory (119) for storing a minimum permissible amplitude difference value for stimulation rate adaptation based on heart signal shape, a variability comparison unit (121) connected to the variation width memory (119) for comparing the actually determined amplitude variability value to the stored minimum amplitude variability value, and a switching unit (115) controllable by the output of the variability comparison unit (121) to transmit a substitute rate-control signal to the stimulation pulse generator (102) if the minimum amplitude variability value is fallen below, the substitute rate-control signal being calculated based on the sensor signal.

12. Rate-adaptive pacemaker according to any one of claims 8 to 11, **characterised in that** the rate calculation device comprises a long-term amplitude difference memory (123), a long-term sensor signal memory (124), and a multistage statistical analysis unit (125), connected to both long-term memories (123, 124) and used to form an amplitude difference histogram and a sensor signal histogram and to establish a correlation between the histograms, the rate-control signal being generated as a function of the established correlation.

13. Rate-adaptive pacemaker according to any one of the preceding claims, **characterised in that** the rate calculation device (100A) comprises an amplitude difference memory or a rate-control signal memory (113), a trend calculation stage (116) connected to the amplitude difference memory or rate-control signal memory (113), a substitute control signal calculation unit (117) connected to the output of the trend calculation stage (116), and a switching unit (115) connected at the input side to the output of the stimulation pulse generator (102) and/or the heart-signal input stage (101) in such a way that if the stimulation pulse generator is blocked or an evoked heart signal is not detected, the rate-control signal is generated by the substitute control signal calculation unit (117) by updating the amplitude difference trend or rate-control signal trend determined by the trend calculation stage (116).

14. Rate-adaptive pacemaker according to any one of the preceding claims, **characterised in that** signal-width detection means (127) for detecting the actual heart signal width, in particular beginning with the emitting of a stimulation pulse or the exceeding of a predetermined signal start amplitude, and ending by falling below a predetermined signal end amplitude are connected downstream of the heart signal input stage (101), and the rate calculation device (100A) comprises a signal-width processing unit (100A.2) for generating the rate control signal based on the actual heart signal width.

15. Rate-adaptive pacemaker according to claim 14, **characterised in that** the signal-width processing unit (100A.2) comprises a signal-width memory (128) connected to the signal-width detection means (127), a calculation unit (129) connected to the signal-width detection means (127) and to the signal-width memory (128), a reference value memory (131), and a comparison unit (130) connected to the calculation unit (129) and the reference value memory (131), the comparison unit comparing a variable dependent on the actual signal width to a reference value, and an AND gate (133) is connected to the outputs of signal-amplitude processing unit (100A.1) and the signal-width processing unit (100A.2), the AND gate permitting the direct output of the rate-control signal generated by the signal-amplitude processing unit (100A.1), provided that the comparison result of the comparison unit has a predetermined quality.

16. Rate-adaptive pacemaker according to claim 15, **characterised in that** the signal-width processing unit (100A.2) comprises a correction value calculation stage (132), of which the output, together with the output of the signal-amplitude processing unit (100A.1), is conveyed to a correction stage (134), which is connected via an inverter (133a) to the output of the AND gate (133) in such a way that if the comparison result does not have the predetermined quality, a correction of the rate-control signal generated by the signal-amplitude processing unit (100A.1) is effected.

## Revendications

1. Stimulateur cardiaque à fréquence adaptative (100) comportant:
un générateur d'impulsions de stimulation (102) et un étage sortie (103) relié à ce générateur et servant à stimuler au moins un ventricule du coeur,
un étage (101) d'entrée du signal cardiaque pour détecter la forme de signaux cardiaques suscités, notamment de la morphologie de la réponse d'excitation ventriculaire suscitée VER,
un dispositif (100A) de calcul de la cadence, qui est relié côté entrée à l'étage (101) d'entrée du signal cardiaque et, côté sortie, au générateur d'impulsions de stimulation, pour le calcul de la cadence de stimulation en fonction de la charge physiologique à laquelle est soumis le porteur du stimulateur cardiaque et pour la délivrance d'un signal de commande de cadence,
dans lequel le dispositif (100A) de calcul de cadence produit le signal de commande de cadence sur la base de la forme du signal cardiaque détectée par l'étage d'entrée,
**caractérisé en ce que**
le dispositif (100A) de calcul de cadence possède une unité (100A.1) de traitement des amplitudes du signal, en réponse à une différence des amplitudes du signal cardiaque entre deux valeurs d'amplitude du signal cardiaque à deux instants d'un cycle cardiaque prédéterminé, notamment du dernier cycle précédent.

2. Stimulateur cardiaque à fréquence adaptative selon la revendication 1, **caractérisé en ce que** l'unité (100A.1) de traitement des amplitudes du signal comporte un étage soustracteur (108) servant à déterminer la différence des amplitudes du signal cardiaque à deux instants prédéterminés du cycle.

3. Stimulateur cardiaque à fréquence adaptative selon la revendication 2, **caractérisé en ce que** l'unité (100A.1) de traitement des amplitudes du signal comporte un dispositif (106) de mémoire du signal cardiaque servant à mémoriser une multiplicité d'allures du signal cardiaque et une unité (107) d'évaluation de variations, qui est reliée au dispositif (106) de mémoire du signal cardiaque et qui détermine, pour un intervalle de temps prédéterminé et un nombre prédéterminé de signaux cardiaques suscités, les deux instants du cycle du signal cardiaque, pour lesquels la différence des amplitudes du signal cardiaque présente une variabilité maximale.

4. Stimulateur cardiaque à fréquence adaptative selon la revendication 3, **caractérisé en ce que** le dispositif (100A) de calcul de cadence comporte une mémoire (112) de valeurs limites de cadence pour une valeur limite inférieure et une valeur limite supérieure de la cadence de stimulation, des moyens (120) de mémoire de différences d'amplitude pour une différence minimale et une différence maximale d'amplitudes du signal cardiaque, et un étage de normalisation (111), qui est relié aux moyens de mémoire de la cadence de stimulation et de la différence d'amplitudes, et qui détermine, pour l'intervalle de temps prédéterminé ou le nombre prédéterminé de signaux cardiaques, une dépendance, normalisée de façon physiologique, de la différence des amplitudes du signal cardiaque par rapport à la cadence adaptée de stimulation, par le fait qu'il combine les différences minimale et maximale mémorisées des amplitudes du signal cardiaque à la valeur limite inférieure et à la valeur limite supérieure mémorisées pour la cadence de stimulation, et qui fournit le signal de commande de cadence à partir d'écarts de la différence réellement déterminée des amplitudes du signal cardiaque vis-à-vis de la dépendance normalisée du point de vue physiologique.

5. Stimulateur cardiaque à fréquence adaptative selon l'une des revendications précédentes, **caractérisé en ce que** l'étage (101) d'entrée du signal cardiaque est agencé pour déterminer au moins l'onde R+ du comportement VER et que le dispositif (100A) de calcul de cadence est agencé de manière à produire le signal de commande de cadence en réponse à une différence des amplitudes du signal cardiaque déterminée dans cette partie du signal cardiaque.

6. Stimulateur cardiaque à fréquence adaptative selon l'une des revendications précédentes, **caractérisé en ce que** les instants prédéterminés du cycle sont maintenus invariables par rapport à des variations de la cadence de stimulation.

7. Stimulateur cardiaque à fréquence adaptative selon l'une des revendications 4 à 6, **caractérisé en ce que** le dispositif (100A) de calcul de la cadence comporte un étage de lissage, qui, en réponse à un écart, qui dépasse une valeur prédéterminée, de la différence réellement déterminée des amplitudes du signal cardiaque par rapport à la dépendance normalisée physiologiquement, fournit un signal de commande de cadence qui réalise une adaptation échelonnée de la cadence de stimulation.

8. Stimulateur cardiaque à fréquence adaptative selon l'une des revendications précédentes, **caractérisé en ce qu'**un détecteur supplémentaire d'activité (S), relié à une entrée du dispositif (100A) de calcul de cadence, est prévue pour la détection d'un paramètre qui dépend de la contrainte physiologique, et que le dispositif (100A) de calcul de cadence comporte des moyens pour prendre en compte le signal du détecteur lors de la production du signal de commande de cadence.

9. Stimulateur cardiaque à fréquence adaptative selon la revendication 8, **caractérisé en ce que** le dispositif (100A) de calcul de cadence comporte une unité (118) de traitement du signal du détecteur, qui détermine pour l'intervalle de temps prédéterminé ou pour le nombre prédéterminé de cycles cardiaques, et ce sur la base de la valeur maximale apparue du paramètre physiologique, la valeur limite supérieure pour la cadence de stimulation.

10. Stimulateur cardiaque à fréquence adaptative selon la revendication 8 ou 9, **caractérisé en ce que** le dispositif (100A) de calcul de cadence comporte des moyens (120) de mémoire de différence des amplitudes pour mémoriser une valeur de différence maximale admissible des amplitudes pour l'adaptation de la cadence de stimulation sur la base de la forme du signal cardiaque, une unité (122) de comparaison de différences des amplitudes reliée aux moyens (120) de mémoire de différence des amplitudes pour comparer la valeur de différence des amplitudes actuellement déterminée à la valeur maximale de différence des amplitudes mémorisée et une unité de commutation (115) commandable par l'intermédiaire de la sortie de l'unité de comparaison de différences des amplitudes pour la transmission d'un signal de commande de cadence de remplacement calculé sur la base du signal du détecteur, au générateur d'amplitude de stimulation (102) lors du dépassement de la valeur maximale de différence des amplitudes.

11. Stimulateur cardiaque à fréquence adaptative selon l'une des revendications 8 à 10, **caractérisé en ce que** le dispositif (100A) de calcul de cadence possède une mémoire (119) de largeur de variation pour mémoriser une valeur de variabilité d'amplitude minimale admissible pour l'adaptation de la cadence de stimulation sur la base de la forme du signal cardiaque, une unité de comparaison de variabilité (120) reliée à la mémoire (119) de largeur de variation pour la comparaison de la valeur de variabilité d'amplitude actuellement déterminée à la valeur minimale de la variabilité d'amplitude mémorisée, et une unité de commutation (115) commandable au moyen de la sortie de l'unité de comparaison de variabilité (121) pour déterminer un signal de commande de cadence de remplacement calculé sur la base du signal de détecteur au générateur d'impulsions de stimulation (102) lorsque la valeur de variabilité d'amplitude tombe au-dessous de sa valeur minimale.

12. Stimulateur cardiaque à fréquence adaptative selon l'une des revendications 8 à 11, **caractérisé en ce que** le dispositif de calcul de cadence comporte une mémoire (123) de différence des amplitudes de longue durée, une mémoire (124) du signal du détecteur de longue durée et un circuit d'évaluation statistique (125) à plusieurs étages, qui est relié aux deux mémoires de longue durée (123, 124) et sert à former un histogramme de la différence des amplitudes et un histogramme du signal du détecteur et à établir une corrélation entre les deux histogrammes, le signal de commande de cadence étant produit en fonction de la corrélation établie.

13. Stimulateur cardiaque à fréquence adaptative selon l'une des revendications précédentes, **caractérisé en ce que** le dispositif (100A) de calcul de cadence comporte une mémoire (113) de différence des amplitudes ou du signal de commande de cadence, un étage de calcul de tendance (116) relié à la mémoire (113) de différence des amplitudes ou de signaux de commande de cadence, et une unité (117) de calcul du signal de commande de remplacement qui est reliée à la sortie de l'étage de calcul de cadence (116), ainsi qu'une unité de commutation (115) qui est reliée côté entrée à la sortie du générateur d'impulsions de stimulation (102) et/ou à l'étage (101) d'entrée du signal cardiaque, de telle sorte que lors de l'inhibition du générateur d'impulsions de stimulation ou de la non détection d'un signal cardiaque suscité, le signal de commande de cadence est produit par l'unité (117) de calcul du signal de commande de remplacement lors de la mise à jour de la tendance du signal de différence des amplitudes ou du signal de commande de cadence déterminé par l'étage de calcul de tendance (116).

14. Stimulateur cardiaque à fréquence adaptative selon l'une des revendications précédentes, **caractérisé en ce qu'**en aval de l'étage (101) d'entrée du signal cardiaque sont branchés des moyens (127) pour la détection de la largeur réelle du signal, notamment en commençant avec la délivrance d'une impulsion de stimulation ou avec le dépassement d'une amplitude initiale prédéterminée du signal et en se terminant par le passage de l'amplitude de fin du signal au-dessous d'une valeur prédéterminée, et que le dispositif (100A) de calcul de cadence comporte une unité (100A.2) de traitement de la largeur du signal, servant à produire le signal de commande de cadence en tenant compte de la largeur du signal cardiaque.

15. Stimulateur cardiaque à fréquence adaptative selon la revendication 14, **caractérisé en ce que** l'unité (100A.2) de traitement de la largeur du signal possède une mémoire de largeur du signal (128) qui est reliée aux moyens (127) de détection de la largeur du signal, une unité de calcul (129) reliée aux moyens (127) de détection de la largeur du signal et à la mémoire (128) de largeur du signal, une mémoire de valeur de référence (131) et une unité de comparaison (131) reliée à l'unité de calcul (129) et à la mémoire de valeur de référence (132) pour comparer une grandeur, qui dépend de la largeur réelle du signal, à une valeur de référence, et qu'aux sorties de l'unité (100A.1) de traitement des amplitudes du signal et de l'unité (100A.2) de traitement de la largeur du signal est reliée une porte ET (133), qui permet la délivrance directe du signal de commande de cadence produit par l'unité (100A.1) de traitement des amplitudes du signal uniquement lorsque le résultat de la comparaison présente une caractéristique prédéterminée.

16. Stimulateur cardiaque à fréquence adaptative selon la revendication 15, **caractérisé en ce que** l'unité (100A.2) de traitement de la largeur du signal comporte un étage (132) de calcul de valeur de correction, dont le signal de sortie est envoyé, conjointement avec le signal de sortie de l'unité (100A.1) de traitement de l'amplitude du signal, à un étage de correction (134), qui est relié par l'intermédiaire d'un inverseur (133a) à la sortie de la porte ET (133) de telle sorte que, lorsque le résultat de la comparaison ne possède pas la caractéristique prédéterminée, une correction du signal de commande de cadence, produit par l'unité (100A.1) de traitement des amplitudes du signal est exécutée.
